(19) Europäisches Patentamt
European Patent Office
Office européen des brevets

(11) **EP 2 326 262 B1**

## (12) EUROPEAN PATENT SPECIFICATION

(45) Date of publication and mention
of the grant of the patent:
**25.03.2015 Bulletin 2015/13**

(51) Int Cl.:
*A61B 17/16* (2006.01)    *A61B 17/295* (2006.01)

(21) Application number: **09749807.5**

(22) Date of filing: **18.05.2009**

(86) International application number:
**PCT/EP2009/056020**

(87) International publication number:
**WO 2009/141317 (26.11.2009 Gazette 2009/48)**

(54) **LAMINECTOMY FORCEPS**

LAMINEKTOMIE-ZANGE

ÉCARTEUR POUR LAMINECTOMIE

(84) Designated Contracting States:
**AT BE BG CH CY CZ DE DK EE ES FI FR GB GR
HR HU IE IS IT LI LT LU LV MC MK MT NL NO PL
PT RO SE SI SK TR**

(30) Priority: **19.05.2008 IT MC20080080**

(43) Date of publication of application:
**01.06.2011 Bulletin 2011/22**

(73) Proprietors:
 • **Melozzi, Alessandro
  64100 Teramo (TE) (IT)**
 • **Magliani, Vincenzo
  64100 Teramo (TE) (IT)**

(72) Inventor: **MAGLIANI, Vincenzo
64100 Teramo (IT)**

(74) Representative: **Baldi, Claudio
Ing. Claudio Baldi S.r.l.
Viale Cavallotti, 13
60035 Jesi (Ancona) (IT)**

(56) References cited:
**GB-A- 918 577      US-A- 5 273 519
US-A- 5 484 441**

## Description

The present invention relates to a laminectomy forceps.

Laminectomy is a surgical operation that provides for total or partial removal of one or more laminae of one or more vertebrae, opening the vertebral canal, to treat spinal pathologies of different nature; laminectomy is used to obtain the excision of the posterior arch of the vertebra.

During laminectomy the surgeon removes bone fragments with a forceps: different types of laminectomy forceps are known at the state of the art, such as Kerrison forceps or Citelli forceps.

In general, all forceps are provided with a handle joined to a fixed beak, with a sliding tray actuated by a lever in opposite position to the handle; the beak has a backing end for the cutting end of the tray in order to generate the cutting area.

The actuation lever is articulated to the tray according to a first-degree lever; more precisely, a pivot is positioned between the handle and the actuation lever and the prosecution of the handle lever, beyond the pivot, towards the tray, is the actuation arm for the tray; the tray is normally provided with an overturned U cross-section, in such a way that an empty area is obtained between the tray and the beak; the handle rests on the palm of the surgeon, who actuates the actuation lever with his fingers.

The bone excision operation is performed in multiple phases, and in each phase the surgeon actuates the forceps to remove a fragment; obviously, this operation must be extremely precise, also because the operation area is dangerously close to the nerve centres of the vertebral column, which could be damaged with pernicious consequences.

The laminectomy forceps of known type are impaired by several drawbacks: firstly, the force exerted by the surgeon on the actuation lever to remove every bone fragment is relatively high and causes early fatigue of the surgeon's hand.

Secondly, the bone fragments removed from the vertebrae get often stuck between the tray and the beak, inside the overturned-U section of the tray, in such a way that their removal slows down the surgical operation and sometimes the forceps must be changed several times during the same surgical phase.

Thirdly, the cutting end of the tray and the cutting ends of the backing border of the beak tend to wear out rapidly, requiring the early replacement of the entire forceps. US 5,484,411 discloses a rongeur surgical instrument defining the technical features of the preamble of claim 1.

The purpose of the present invention is to solve the aforementioned inconveniences with a surgical forceps for laminectomy as claimed in the first claim.

Further advantageous characteristics are the subject of the enclosed claims.

The present invention is defined by claim 1 and provides for multiplying the force exerted by the surgeon on the actuation lever by means of a force multiplier lever mechanism, in such a way to prevent the surgeon's early fatigue, and increase the speed and accuracy of the surgeon's action.

Secondly, the forceps according to the present invention comprises a cleaning device with sliding rod inside the tray, in such a way to remove the bone fragments that get stuck in the tray.

Thirdly, the forceps as claimed in the present invention allows for disassembling the cutting parts when they are worn out in order to replace them when necessary, without changing the entire forceps.

Additional characteristics and advantages of the present invention will appear more evident from the following description and enclosed drawings, which have only an illustrative, not limiting purpose, wherein:

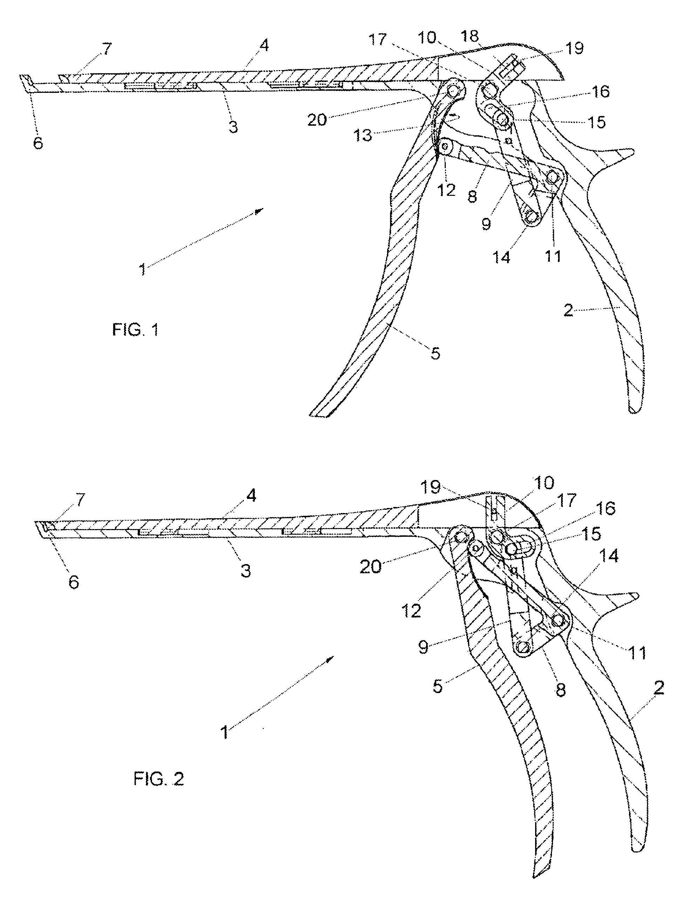

> fig. 1 is a sectional view of a laminectomy forceps according to the present invention in open condition;
> fig. 1 is a sectional view of a laminectomy forceps according to the present invention in closed condition;
> fig. 1 is a lateral view of a laminectomy forceps according to the present invention in open condition;
> fig. 1 is a lateral view of a laminectomy forceps according to the present invention in closed condition;
> fig. 5 is a sectional view of the tray of the laminectomy forceps according to the present invention provided with cleaning device;
> fig. 6 is a top view of the tray of the laminectomy forceps according to the present invention provided with cleaning device;
> fig. 7 is a cross-sectional view of the tray of the laminectomy forceps according to the present invention provided with cleaning device;
> figs. 8A and 8B are enlarged views of fig. 3 and fig. 4.

Figs. 1 and 2 show a laminectomy forceps (1) comprising: a handle (2) joined to a fixed beak (3) with a sliding tray (4) actuated by an actuation lever (5) in opposite position to the handle (2), in such a way that the surgeon places the handle (2) on his palm and actuates the actuation lever (5) with the fingers of the same hand.

The fixed beak (3) is provided with a backing end (6) that acts as stop for the cutting end (7) of the tray (4), in such a way to generate the cutting area in which the surgeon places the part of bone to be removed.

The forward movement of the tray (4) towards the backing end (6) determines the cut and excision of the bone fragment included between them by means of the cutting edges situated in opposite position on the cutting end (7) and the backing end (6).

The actuation lever (5) according to the precepts of the present invention is articulated with the tray (4) by means of a lever mechanism designed to multiply the force exerted by the surgeon on the actuation lever (5) and transmit the multiplied force to the tray (4).

According to the preferred embodiment illustrated in the enclosed figures the mechanism lever is composed of three arms: a first arm (8) hinged on the handle (2), which is articulated to a second arm (9), which is in turn articulated with a third arm (10) that actuates the tray (4), as explained below.

The first arm (8) is hinged at one end to the handle (2) with the first pin (11) and is provided at the opposite end with a roll (12) free to slide in the inlet (13) obtained on the internal side of the actuation lever (5): further to the forward movement of the actuation lever (5) towards the handle (2), the roll (12) moves in the inlet between two stop positions, the first one illustrated in fig. 1 and the second one in fig. 2, thus determining the rotation of the first arm (8) around the first pin (11).

The first arm (8) has an L-shape and its free end in opposite position to the roll (12) is hinged with the second pin (14) to the second arm (9), thus moving it consequently.

The free end of the second arm (9) opposite to the end hinged with the first arm (8) is provided with a third pin (15) that slides inside the slot (16) of the third arm (10), which has an L-shape and is hinged to the handle (2) with the fourth pin (17).

The free end of the third arm (10) in opposite position to the slot (16) is provided with a sliding guide (18) for the constraint (19), which is joined with the tray (4) that can only slide forward and backward on the fixed beak (3).

Obviously, also the actuation lever (5) is hinged on the handle (2) by means of the pin (20).

The operation of the forceps (1) according to the present invention is shown in figs. 1 and 2, which illustrate the two extreme conditions of open and closed forceps: when the surgeon actuates the actuation lever (5) by moving it closer to the handle (2), the roll (12) slides on the inlet (13) and the first arm (8) rotates around the first pin (11); the opposite end of the first arm (8) actuates by means of the second pin (14) on the second arm (9) that moves, making the third pin (15) slide inside the slot (16), thus actuating the third arm (10) in rotation around the fourth pin (17); the fourth arm actuates on the constraint (19) by means of the sliding guide (18) in which the constraint (19) moves, driving the tray (4) closer to the cutting end (7) towards the backing end (6).

In the aforementioned configuration the lever mechanism used to actuate the tray (4) comprises two arms (8, 10) that are both hinged on the handle (2) and mutually connected by an additional arm (9).

The following calculation is given for merely illustrative purposes: with the following dimensions:

- length of actuation lever (5) = 115.86 mm = A;
- distance between the axis of the actuation pin (20) and the axis of the roll (12) with forceps in closed condition = 3.21 mm = B;
- distance between the axis of the first pin (11) and the axis of the third pin (15) with forceps in closed condition = 26.05 mm = C;
- distance between the axis of the first pin (11) and the axis of the second pin (14) with forceps in closed condition = 15.43 mm = D;
- distance between the axis of the second pin (14) and the axis of the fourth pin (17) with forceps in closed condition = 39.63 mm = E;

a load $F_1$ = 5 kg applied at the end of the actuation lever (5) determines a load $F_2$ exerted on the tray by the constraint (19) equal to:

$$F_2 = F_1 * (A/B * C/D * E/F) = 1280 \text{ kg}.$$

The above clearly shows the advantages of the said lever mechanism, since the force transmitted by the handle lever to the tray can be hardly obtained using the principle of first degree lever, such in the case of the known forceps, in which the forceps should be dimensioned in such a way to prevent the correct use in the surgical field and/or use with two hands.

With the forceps according to the present invention the surgeon can advantageously avoid early fatigue of the hand, dosing the force correctly and being more accurate in his action.

Alternatively, although with a lower force ratio, the third arm (10) can be eliminated and the second arm (9) can be extended until it actuates on the constraint (19) of the tray.

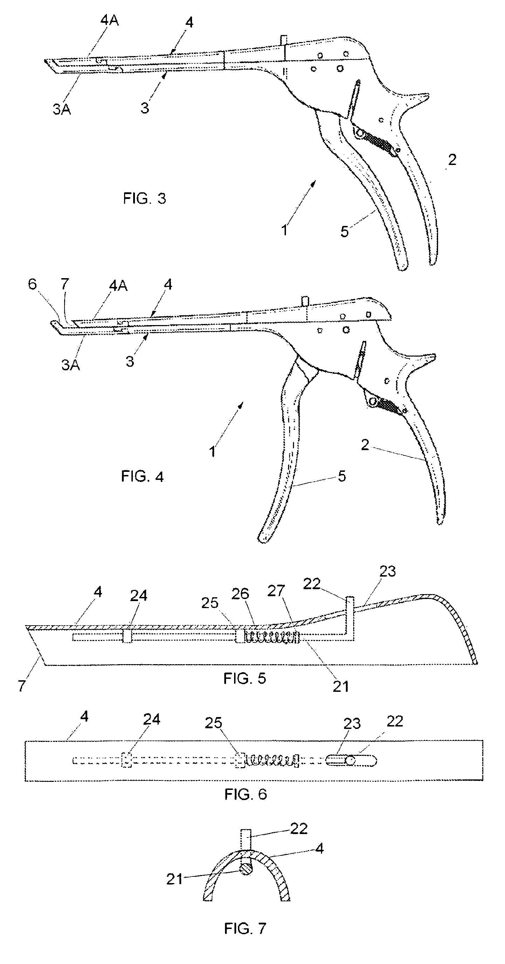

An additional advantageous characteristic of the forceps is illustrated in figs. 3 and 4, in which both the tool-holder portion (3A) of the beak (3) with the cutting backing end (6) and the tool-holder portion (4A) of the tray (4) with the cutting end (7) are of removable type.

Basically, the tool-holder portion (3A) of the fixed beak (3) is a separate part of the beak (3) and is joined to the beak (3) by means of screws or similar removable connection means that allow for replacement when the cutting edges are worn out.

Similarly, also the tool-holder portion (4A) of the tray (4) is a separate part of the tray (4) and is joined to the tray (4) by means of screws or similar removable connection means that allow for replacement when the cutting edges are worn out.

Advantageously, this allows for replacing only the cutting edges when they have lost sharpening due to extended use, without having to replace the entire forceps, as in the case of known models of forceps.

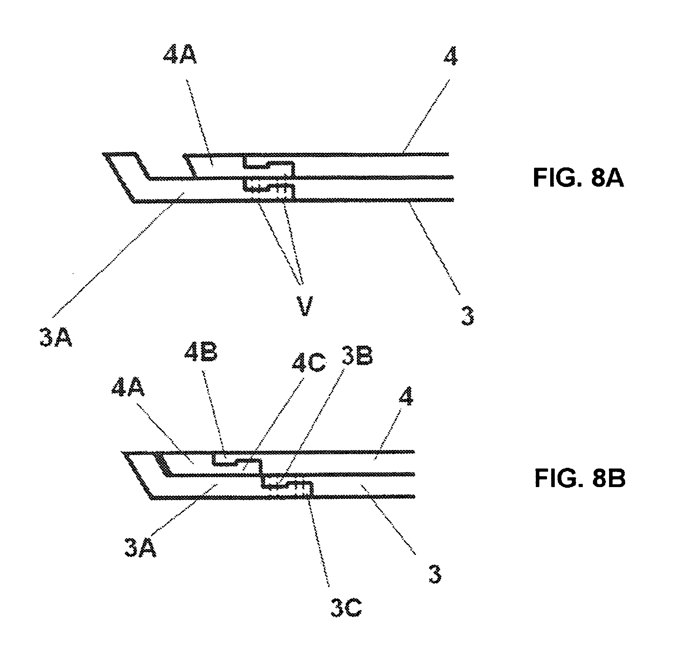

According to a preferred embodiment (shown in figs. 8A and 8B), the tool-holder portions (4A, 3A) are engaged on the tray (4) and on the fixed beak (3), respectively, as illustrated, with double-hook engagement means (3B, 3C and 4B, 4C) for rapid assembly; in this case, a simple analysis of the figures demonstrates the operation principle: when the forceps is in disassembled condition (that is to say without the tool-holder end 4A, 3A), the forceps is actuated in closed condition, in such a way that the tray (4) moves forward until the section with downward-facing hooked profile (4b) protrudes from the fixed beak (3), and then the tool-holder portion (4A) is inserted and the actuation lever is released, making the tray (4) move backwards towards the handle (2).

This causes the locking in position of the tool-holder portion (4A) also without screws and without positioning the tool-holder portion (3A) of the fixed beak (3): when the forceps is opened, the tool-holder portion (4A) rests under the fixed beak (3) and is prevented on top from being released from the double-hook profile (4B and 4C).

Then the forceps is completed by positioning the tool-holder portion of the fixed beak (3A) in the double-hook seat (3B and 3C) and by fixing it to the fixed beak (3) with screws.

Not only this allows for replacing the cutting edges when they are worn out, but also to replace them quickly, since they are both fixed in operating position with only one screw.

Figs. 5, 6 and 7 illustrate an additional advantageous characteristic of the forceps, that is to say the cleaning device.

The cleaning device is basically composed of a rod (21) that slides inside the tray (4), which is provided with overturned U cross-section.

The rod can be actuated manually with a control handle (22), which is shown in an illustrative form as a transversal extension of the rod (21), sliding in a slot (23) obtained in upper position on the body of the tray (4).

The rod (21) slides inside the tray (4) in such a way to move forward towards the cutting end (7) and free the tray (4) from the bone fragments that can be stuck therein; the rod (21) slides in a sliding guide that is illustratively shown as obtained with two sliding eyelets (24, 25).

According to another advantageous characteristic the rod (21) is provided with autonomous return means, such as a spring (27) that actuates on an eyelet (25) and on a projecting ridge (27) of the rod (21).

Additional variations and additions to the forceps according to the present invention can be provided without leaving the precepts and the purpose of the present invention.

For example, automatic return means to open position of the forceps can be provided, such as a spring, either helicoidal or obtained with two pivoting metal plates in opposite position (being one fixed to the actuation lever and the other one to the handle), designed to push the actuation lever firmly away from the handle.

Moreover, autonomous return means of the cleaning rod that differ from the ones illustrated and described herein can be provided, or alternatively the sliding guide of the rod can have different shapes.

Although according to the illustrated forceps the cutting end (7) and the relevant backing end (6) are in oblique position with respect to the sliding direction of the tray (4), it is possible to provide them in perpendicular position.

An additional alternative embodiment, which is not illustrated in the enclosed figures, provides that the relative positions of the tray and the fixed beak are reversed, in such a way that the cutting area faces downwards instead of upwards, as shown.

The said alternative can be useful when the point of the operation is especially troublesome or not easily accessible with a forceps as the illustrated one.

In such a case, by applying the aforementioned precepts, the mutual positions of the tray (4) and the fixed beak (3) can be exchanged, in such a way that the fixed beak (3) is situated above the tray (4), which faces the actuation lever (5).

## Claims

1. A laminectomy forceps (1) comprising:

a handle (2) joined to a fixed beak (3) having a sliding tray (4) defining a cutting end (7);
an actuation lever (5) in opposite position of the handle for actuating the sliding tray (4);
wherein the fixed beak (3) is provided with a cutting backing end (6) corresponding to the cutting end (7) of the tray (4) in order to generate a cutting area;
a force multiplier lever mechanism (8, 9, 10); and
wherein the actuation lever (5) is adapted to articulate with respect to the tray (4) and is connected thereto by the force multiplier lever mechanism such that the actuation lever (5) determines sliding of said tray (4);
**characterised in** the force multiplier lever mechanism (8, 9, 10) comprising:

a first L-shaped arm (8) hinged to the handle and arranged to be actuated by said lever (5);
a second arm (9) connected to and configured to articulate with respect to the first L-shaped arm (8);
a third L-shaped arm (10) connected to and configured to articulate to the second arm (9), the third L-shaped arm (10) being hinged to the handle (2) and arranged to actuate the tray (4);
wherein the first L-shaped arm defines a longer portion configured to interact with the actuation lever (5) and a shorter portion connected to said second arm (9);
wherein the third L-shaped arm defines a shorter portion connected to the second arm and a longer portion connected to the tray (4); and

wherein the arms are arranged so that said force multiplier lever mechanism multiply the force exerted by a surgeon on the actuation lever (5) and transmit the multiplied force to the tray (4).

2. A laminectomy forceps as defined by claim 1, **characterised in that** the first L-shaped arm (8) is hinged at one end to the handle (2) by means of a first pin (11) and is provided at the opposite end with a roll (12) free to slide in the inlet (13) obtained on the internal side of the actuation lever (5), and in which the free end of the first L-shaped arm (8) opposite to the end with the roll (12) is hinged with a second pin (14) to the second arm (9) with free end opposite to the end hinged to the first L-shaped arm (8), comprising a third pin (15) that slides inside a slot (16) provided on the third L-shaped arm (10) and in which the free end of the third L-shaped arm (10) in opposite position to the slot (16), is provided with a sliding guide (18) for a constraint (19) joined to the tray (4) in order to make the tray (4) slide with respect to the fixed beak (3).

3. A laminectomy forceps as defined by claim 1 or 2, **characterised in that** the actuation lever (5) is hinged on the handle (2) by means of an actuation pin (20).

4. A laminectomy forceps as defined by any preceding claim, **characterised in that** the fixed beak (3) comprises a tool-holder portion (3A) with the cutting backing end (6), the said tool-holder portion (3A) being removably fixed with respect to the fixed beak (3) and **in that** the tray (4) comprises a tool-holder portion (4A) with the cutting end (7), the said tool-holder portion (4A) being removably fixed with respect to the tray (4).

5. A laminectomy forceps as defined by claim 4, **characterised in that** the tool-holder portion (3A) of the fixed beak is fixed removably by means of detachable connection means, such as screws or similar means.

6. A laminectomy forceps as defined by claims 4 or 5, **characterised in that** the tool-holder portions (4A, 3A) are respectively engaged on the tray (4) and on the fixed beak (3) with double-hook engagement means (3B, 3C and 4B, 4C), with the tray (4) protruding from the fixed beak (3) when the laminectomy forceps is closed and the tool-holder portions (3A and 4A) are disassembled, in such a way to lock in position the tool-holder portion (4A) of the tray when the laminectomy forceps is open without assembling the tool-holder portion (3A) of the fixed beak (3).

7. A laminectomy forceps as defined by any preceding claim, **characterised in that** it comprises a cleaning, device to clean the tray (4).

8. A laminectomy forceps as defined by claim 7, **characterised in that** the tray (4) has an overturned U cross-section and the cleaning device comprises a rod (21) that slides inside the tray (4).

9. A laminectomy forceps as defined by claim 8, **characterised in that** the rod (21) is actuated by means of a control handle (22) in transversal position to the rod (21), with the control handle (22) sliding in a slot (23) obtained in upper position on the body of the tray (4).

10. A laminectomy forceps as defined by claim 7 or 8, **characterised in that** the rod (21) is configured to slide inside a sliding guide that comprises at least one sliding eyelet (24, 25).

11. A laminectomy forceps as defined by any one of claims 7 to 10, **characterised in that** the rod (21) is provided with autonomous return means, such as a spring (27) or similar means.

12. A laminectomy forceps as defined by any preceding claim, **characterised in that** it also comprises automatic return means to open position of the laminectomy forceps, such as a helical spring or two pivoting metal plates in opposite position one being fixed to the actuation lever and the other one to the handle, designed to push the actuation lever firmly away from the handle.

**Patentansprüche**

1. Chirurgische Zange für Laminektomie (1) umfassend:

- einen Griff (2), der fest mit einem festen Maul (3) mit einem Gleiteinsatz (4) verbunden ist, der ein schneidendes Ende (7) definiert,
- einen dem Griff gegenüberliegenden Betätigungshebel (5), um den Gleiteinsatz (4) zu betätigen;
wobei das feste Maul (3) ein schneidendes Anschlagende (6) als Anschlag für das schneidende Ende (7) des Gleiteinsatzes (4) aufweist, so dass eine Schneidezone erzeugt wird;
- einen Hebelmechanismus zur Kraftverstärkung (8, 9, 10),
wobei der Betätigungshebel (5) dazu geeignet ist, am Einsatz (4) angelenkt und mit diesem über den Hebelmechanismus zur Kraftverstärkung verbunden zu werden, damit der Betätigungshebel ein Gleiten des Einsatzes (4) bewirkt;

**dadurch gekennzeichnet, dass** der Hebelmechanismus zur Kraftverstärkung Folgendes umfasst:

- einen ersten L-förmigen Arm (8), der am Griff angelenkt und entsprechend angeordnet ist, um von dem Betätigungshebel (5) gelenkig bewegt zu werden;
- einen zweiten Arm (9), der mit dem ersten, L-förmigen Arm (8) verbunden und entsprechend gestaltet ist, um gelenkig gegenüber dem ersten L-förmigen Arm (8) bewegt zu werden;
- einen dritten L-förmigen Arm (10), der mit dem zweiten Arm (9) verbunden und entsprechend gestaltet ist, um gegenüber dem zweiten Arm (9) gelenkig bewegt zu werden, wobei der dritte L-förmige Arm (10) am Griff (2) angelenkt und entsprechend angeordnet ist, um den Einsatz (4) zu betätigen;

wobei der der erste L-förmige Arm einen längeren Anteil definiert, der entsprechend gestaltet ist, um mit dem Betätigungshebel (5) zusammenzuwirken, und einen kürzeren Anteil, der mit dem zweiten Arm (9) verbunden ist;

wobei der dritte L-förmige Arm einen kürzeren Anteil definiert, der mit dem zweiten Arm (9) verbunden ist, und einen längeren Anteil, der mit dem Einsatz (4) verbunden ist;

wobei die Arme derart angeordnet sind, dass der Hebelmechanismus zur Kraftverstärkung die von einem Chirurgen auf den Betätigungshebel (5) ausgeübte Kraft verstärkt und die verstärkte Kraft auf den Einsatz (4) überträgt.

2. Chirurgische Zange für Laminektomie nach Anspruch 1, **dadurch gekennzeichnet, dass** der erste L-förmige Arm (8) mit einem seiner Enden mittels eines ersten Bolzens (11) am Griff (2) angelenkt und am entgegengesetzten Ende mit einer frei gleitbaren Rolle (12) versehen ist, die in eine Einbuchtung (13) gleitet, die auf der Innenseite des Betätigungshebels (5) herausgearbeitet ist, wobei das freie Ende des ersten L-förmigen Arms (8), das sich gegenüber demjenigen mit der Rolle (12) befindet, über einen zweiten Bolzen (14) am zweiten Arm (9) angelenkt ist, dessen freies Ende demjenigen gegenüberliegt, an dem der erste L-förmige Arm (8) angelenkt ist, umfassend einen dritten Bolzen (15), der in einem Langloch (16) gleitet, das auf dem dritten L-förmigen Arm (10) vorgesehen ist, wobei das freie Ende des dritten, L-förmigen Arms (10), das dem Langloch (16) gegenüberliegt, eine Gleitführung (18) für eine Blockierung (19) aufweist, die derart mit dem Einsatz (4) verbunden ist, dass dieser gegenüber dem festen Maul (3) gleitend betätigt wird.

3. Chirurgische Zange für Laminektomie nach Anspruch 1 oder 2, **dadurch gekennzeichnet, dass** der Betätigungshebel (5) am Griff (2) mittels eines Betätigungsbolzens (20) angelenkt ist.

4. Chirurgische Zange für Laminektomie nach einem beliebigen der vorstehenden Ansprüche, **dadurch gekennzeichnet, dass** das feste Maul (3) einen Werkzeughalteranteil (3A) mit dem schneidenden Anschlagende (6) umfasst, wobei der Werkzeughalteranteil (3A) abnehmbar gegenüber dem festen Maul (3) befestigt ist, sowie dadurch, dass der Einsatz (4) einen Werkzeughalteranteil (4A) mit dem schneidenden Ende (7) umfasst, wobei der Werkzeughalteranteil (4A) abnehmbar gegenüber dem Einsatz (4) befestigt ist.

5. Chirurgische Zange für Laminektomie nach Anspruch 4, **dadurch gekennzeichnet, dass** der Werkzeughalteranteil (3A) des festen Mauls abnehmbar mittels demontierbarer Verbindungen wie Schrauben o.ä. befestigt ist.

6. Chirurgische Zange für Laminektomie nach Anspruch 4 oder 5, **dadurch gekennzeichnet, dass** die Werkzeughalteranteile (4A, 3A) den Einsatz (4) bzw. das feste Maul (3) mit Eingreifmitteln mit Doppelhaken (3B, 3C und 4B, 4C) in Eingriff nehmen, wobei der Einsatz (4), der bei geschlossener Zange und demontierten Werkzeughalteranteilen (3A und 4A) aus dem festen Maul (3) derart auskragt, dass der Werkzeughalteranteil (4A) des Einsatzes bei geöffneter Zange einrastet, ohne dass der Werkzeughalteranteil (3A) des festen Mauls (3) demontiert werden muss.

7. Chirurgische Zange für Laminektomie nach einem oder mehreren der vorstehenden Ansprüche, **dadurch gekennzeichnet, dass** sie eine Reinigungsvorrichtung zur Reinigung des Einsatzes (4) umfasst.

8. Chirurgische Zange für Laminektomie nach Anspruch 7, **dadurch gekennzeichnet, dass** der Einsatz (4) einen Querschnitt in Form eines umgekehrten U aufweist und die Reinigungsvorrichtung einen Stab (21) umfasst, der im Innern des Einsatzes (4) gleitet.

9. Chirurgische Zange für Laminektomie nach Anspruch 8, **dadurch gekennzeichnet, dass** der Stab (21) mittels eines Bedienungsgriffs (22) betätigt wird, der quer zum Stab (21) liegt, wobei der Bedienungsgriff (22) in einem Langloch (23) gleitet, das oberhalb des Körpers des Einsatzes (4) herausgearbeitet ist.

10. Chirurgische Zange für Laminektomie nach Anspruch 7 oder 8, **dadurch gekennzeichnet, dass** der Stab (21) entsprechend gestaltet ist, um in einer Gleitführung zu gleiten, die mindestens eine Gleitö-

se (24, 25) umfasst.

**11.** Chirurgische Zange für Laminektomie nach einem oder mehreren der Ansprüche von 7 bis 10, **dadurch gekennzeichnet, dass** der Stab (21) mit Mitteln zur selbsttätigen Rückkehr in die Position, beispielsweise eine Feder (27) o.ä. versehen ist.

**12.** Chirurgische Zange für Laminektomie nach einem oder mehreren der vorstehenden Ansprüche, **dadurch gekennzeichnet, dass** sie ferner Mittel zur automatischen Rückkehr in die geöffnete Zangenstellung wie beispielsweise eine Schraubenfeder oder zwei drehbare, gegenüberliegende Metallstreifen umfasst, die zum einen am Betätigungshebel und zum anderen am Griff befestigt und dazu geeignet sind, den Betätigungshebel dauerhaft in die vom Griff entfernte Stellung zu drücken.

## Revendications

**1.** Pince chirurgicale pour laminectomie (1) comprenant :

- une poignée (2) solidaire à un bec fixe (3), ayant un chariot coulissant (4) qui définit une partie terminale coupante (7) ;
- un levier d'actionnement (5) opposé à la poignée pour activer le chariot coulissant (4) ; où le bec fixe (3) présente une partie terminale de butée (6) coupante, de butée pour la partie terminale coupante (7) du chariot (4), de façon à générer une zone de coupure ;
- un mécanisme de levier multiplicateur de force (8, 9, 10), et où le levier d'actionnement (5) est apte à être articulé par rapport au chariot (4) et est connecté moyennant le mécanisme de levier multiplicateur de force, de façon que le levier d'actionnement (5) détermine le coulissement du chariot (4) ;

**caractérisée en ce que** le mécanisme de levier multiplicateur de force (8, 9, 10) comprend :

- un premier bras en « L » (8) articulé sur la poignée et disposé pour être activé par ledit levier d'actionnement (5) ;
- un deuxième bras (9) relié au premier bras en « L » (8) et configuré pour être articulé par rapport au premier bras en « L » (8) ;
- un troisième bras en « L » (10) relié au deuxième bras (9) et configuré pour être articulé par rapport au deuxième bras (9), le troisième bras en « L » (10) étant articulé à la poignée (2) et disposé pour actionner le chariot (4) ; où le premier bras en « L » définit une portion plus longue configurée pour interagir avec le levier d'actionnement (5) et une portion plus courte reliée au dit deuxième bras (9) ; où le troisième bras en « L » définit une portion plus courte reliée au deuxième bras et une portion plus longue reliée au chariot (4) ; et où les bras sont disposés de sorte que ledit mécanisme de levier multiplicateur de force multiplie la force exercée par un chirurgien sur le levier d'actionnement (5) et transmettent la force multipliée au chariot (4).

**2.** Pince chirurgicale pour laminectomie, selon la revendication 1, **caractérisée en ce que** le premier bras en « L » (8) est articulé à l'une de ses extrémités à la poignée (2) moyennant un premier pivot (11) et présente à l'extrémité opposée un rouleau (12) libre de glisser dans une baie (13) réalisée sur la face interne du levier d'actionnement (5), et où l'extrémité libre du premier bras en « L » (8), opposée à celle dotée du rouleau (12), est articulée moyennant un deuxième pivot (14) au deuxième bras (9), qui présente l'extrémité libre opposée à celle articulée avec le premier bras en « L » (8), comprenant un troisième pivot (15) coulissant à l'intérieur d'une fente (16) prévue sur le troisième bras en « L » (10) et où l'extrémité libre du troisième bras en « L » (10), opposée à la fente (16), présente un rail de coulissement (18) pour une contrainte (19), solidaire avec le chariot (4), de manière à actionner le chariot (4) en coulissement par rapport au dit bec fixe (3).

**3.** Pince chirurgicale pour laminectomie, selon les revendications 1 ou 2, **caractérisée en ce que** le levier d'actionnement (5) est articulé sur la poignée (2), au moyen d'un pivot d'actionnement (20).

**4.** Pince chirurgicale pour laminectomie, selon l'une quelconque des revendications précédentes, **caractérisée en ce que** le bec fixe (3) comprend une portion porte-outil (3A) qui supporte la partie terminale de butée (6) coupante, ladite portion porte-outil (3A) étant fixée de manière amovible par rapport au dit bec fixe (3) et **caractérisée en ce que** le chariot (4) comprend une portion porte-outil (4A) qui supporte la partie terminale coupante (7), ladite portion porte-outil (4A) étant fixée de manière amovible par rapport au dit chariot (4).

**5.** Pince chirurgicale pour laminectomie, selon la revendication 4, **caractérisée en ce que** ladite portion porte-outil (3A) du bec fixe est fixée de manière amovible moyennant des connexions démontables, telles que vis ou similaires.

**6.** Pince chirurgicale pour laminectomie, selon les revendications 4 ou 5, **caractérisée en ce que** lesdites portions porte-outil (4A, 3A) sont respectivement en-

gagées sur le chariot (4) et sur le bec fixe (3), avec des moyens d'engagement à double accrochage (3B, 3C et 4B, 4C), le chariot (4) faisant en outre saillie du bec fixe (3) en état de pince fermée et portions porte-outil (3A et 4A) démontées, de façon à bloquer sur la position la portion porte-outil (4A) du chariot en état de pince ouverte et sans que la portion porte-outil (3A) du bec fixe (3) soit montée.

7. Pince chirurgicale pour laminectomie, selon l'une ou plusieurs des revendications précédentes, **caractérisée en ce qu'**elle comprend un dispositif de nettoyage pour l'assainissement dudit chariot (4).

8. Pince chirurgicale pour laminectomie, selon la revendication 7, **caractérisée en ce que** ledit chariot (4) présente une section transversale en forme de « U » renversé et ledit dispositif de nettoyage comprend une tige (21) coulissante à l'intérieur du chariot (4).

9. Pince chirurgicale pour laminectomie, selon la revendication 8, **caractérisée en ce que** ladite tige (21) est actionnée moyennant une poignée de commande (22), transversale à la tige (21), ladite poignée de commande (22) étant coulissante dans une fente (23) réalisée supérieurement sur le corps du chariot (4).

10. Pince chirurgicale pour laminectomie, selon les revendications 7 ou 8, **caractérisée en ce que** ladite tige (21) glisse dans un rail de coulissement comprenant au moins un oeillet de coulissement (24, 25).

11. Pince chirurgicale pour laminectomie, selon l'une ou plusieurs des revendications de 7 à 10, **caractérisée en ce que** la tige (21) est dotée de moyens de retour autonomes, comme par exemple un ressort (27) ou similaires.

12. Pince chirurgicale pour laminectomie, selon l'une ou plusieurs des revendications précédentes, **caractérisée en ce qu'**elle comprend également des moyens de retour automatique en position ouverte de la pince, tel qu'un ressort hélicoïdal ou deux lames métalliques pivotantes et opposées fixées l'une au levier d'actionnement et l'autre à la poignée, aptes à pousser le levier d'actionnement stablement en position d'éloignement de la poignée.

FIG. 1

FIG. 2

FIG. 3

FIG. 4

FIG. 5

FIG. 6

FIG. 7

FIG. 8A

FIG. 8B

**EP 2 326 262 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5484411 A